# FASCICULE DE BREVET EUROPEEN

(11) **EP 0 783 566 B1**
(45) Date de publication et mention de la délivrance du brevet: **04.11.1998**
(21) Numéro de dépôt: 95932806.3
(22) Date de dépôt: 28.09.1995
(51) Int. Cl.: C12N 1/20, A23C 9/123

(54) **SOUCHE DE STREPTOCOCCUS THERMOPHILUS, PROCEDE DE FERMENTATION METTANT EN OEUVRE CETTE SOUCHE ET PRODUIT OBTENU**
STREPTOCOCCUS THERMOPHILUS STAMM, FERMENTATIONSVERFAHREN UNTER VERWENDUNG DIESES STAMM UND DIE HERGESTELLTEN PRODUKTEN
STREPTOCOCUS THERMOPHILUS STRAIN, FERMENTATION PROCESS USING SUCH STRAIN AND PRODUCT OBTAINED

(30) Priorité: 30.09.1994 FR 9411722
(43) Date de publication de la demande: 16.07.1997
(73) Titulaire: COMPAGNIE GERVAIS DANONE, 92300 Levallois Perret (FR)
(72) Inventeur: BENBADIS, Laurent, F-92160 Antony (FR); OUDOT, Elisabeth, F-91570 Bièvres (FR); DE VILLEROCHE, Jacques, F-78000 Versailles (FR)
(74) Mandataire: Warcoin, Jacques
(86) Numéro de dépôt international: FR9501254
(87) Numéro de publication internationale: WO9610627

(56) Documents cités:
- DE-A- 3 300 123
- LE LAIT, vol. 71, no. 4, ELSEVIER, pages 445-461, A. ZOURARI ET AL. 'Caractérisation de bactéries lactiques thermophiles isolées de yaourts artisanaoux grecs. I. Souches de Streptococcus salivarius subsp thermophilus.'
- DATABASE WPI Section Ch, Week 8824 Derwent Publications Ltd., London, GB; Class D16, AN 88-166505 & SU,A,1 351 973 (DAIRY IND. RES. INST.) , 15 Novembre 1987

## Description

La présente invention concerne une souche *Streptococcus thermophilus,* l'utilisation de cette souche pour l'obtention de produits laitiers fermentés et les produits obtenus par la mise en oeuvre de cette souche.

Le choix des bactéries lactiques pour la production de produits laitiers fermentés fait intervenir différents critères, notamment l'activité acidifiante et la formation de composants aromatiques qui assurent les propriétés organoleptiques du produit et la production d'agents épaississants qui jouent un rôle sur la texture et l'onctuosité.

L'activité acidifiante se caractérise essentiellement par trois paramètres : la cinétique d'acidification, l'acidité titrable ou le pH final de fermentation qui conditionne les caractères organoleptiques du produit et son aptitude à la conservation, et la post-acidification qui se développe au cours de la conservation du produit.

Une vitesse d'acidification élevée permet de réduire la période durant laquelle la préparation à base de lait est sensible aux contaminants (pH > 4,7) et de diminuer ainsi le risque de contamination bactérienne.

L'augmentation de la vitesse d'acidification améliore également l'économie du procédé en augmentant la productivité et la flexibilité du matériel industriel.

Les propriétés de post-acidification des souches sont particulièrement importantes pour la conservation des produits. En effet, les produits frais fermentés sont conservés à des températures comprises entre environ 4°C et 8°C pendant une durée n'excédant pas, en général, 4 semaines ; mais, si l'activité métabolique des bactéries est réduite par la conservation au froid, elle n'est pas bloquée et entraîne la production d'acide lactique à partir du lactose, ce qui a pour conséquence une diminution du pH et une augmentation de la saveur acide qui dégradent les propriétés organoleptiques du produit.

Outre les critères retenus pour leur contribution à la qualité du produits, d'autres éléments plus spécifiquement liés au procédé interviennent dans le choix des souches comme la température de fermentation, la vitesse d'acidification et la résistance aux phages.

La résistance aux phages est un critère très important dans le choix des souches pour diminuer le risque d'incidents phagiques en production, susceptibles de bloquer plus ou moins longtemps l'ensemble de la production pour décontamination.

On ne connaît pas à ce jour de souche de *S. thermophilus* qui présente une vitesse d'acidification et un degré d'acidité élevés, associés à une activité post-acidifiante très faible ou quasiment nulle.

En effet, on observe d'une façon générale que les cultures de *S. thermophilus* qui ont une activité de post-acidification réduite montrent souvent une activité acidifiante limitée pendant la fermentation, ce qui ne permet pas d'atteindre le degré d'acidité souhaité dans les produits. En outre, ces souches ont souvent une croissance lente, elles nécessitent des taux d'inoculation élevés et une durée d'incubation plus longue qui sont incompatibles avec l'économie d'un procédé industriel.

La présente invention a pour objet une souche de *S. thermophilus* qui présente une cinétique d'acidification rapide permettant d'atteindre un degré d'acidité élevé et qui ne post-acidifie pas au cours de la conservation des produits frais fermentés.

Plus particulièrement, la présente invention concerne une souche de *S. thermophilus* DN-001 116 déposée à la Collection Nationale de Cultures de Microorganismes (CNCM) sous le n° 1-1477 et ses mutants ayant des caractéristiques d'acidification du lait similaires.

Par souches mutantes ayant des caractéristiques acidifiantes du lait similaires à la souche déposée, on entend désigner des souches qui peuvent être obtenues notamment par mutation et sélection à partir de la souche de référence et/ou par transformation génétique à l'aide de vecteurs.

Les caractéristiques acidifiantes sont un pH de fin d'incubation de 4,6 en moins de 4 heures et une variation de pH à 28 jours de de moins de 0,2 unité pH, de préférence moins de 0,1 unité.

Les souches mutantes peuvent être obtenues à partir de la souche de référence, notamment par mutation et sélection selon leurs propriétés acidifiantes. Les techniques de mutation sont connues, de même que les tests permettant de contrôler les propriétés acidifiantes ( notamment Spinnier H.E., Corrieu G. "Automatic method to quantify starter activity based on pH measurement" J. of Dairy Research, 56 (1989) 755-764) ; de Roissart H. et Luquet F.M. Edition Lorica ISBN : 2-9507477-0-1).

Ces souches sont plus particulièrement intéressantes pour la préparation de produits lactés fermentés de tous types.

C'est pourquoi, la présente invention concerne un procédé de préparation de produits laitiers fermentés dans lequel un substrat lacté est mis en fermentation avec au moins une souche de *S*. *thermophilus.*

Il est également possible de prévoir dans ce procédé l'utilisation d'une association de une ou plusieurs souches bactériennes, notamment avec d'autres bactéries lactiques telles que *Lactobacillus bulgaricus, Lactobacillus acidophilus, Lactobacillus casei* ou *Bifidobacterium.*

De préférence, le substrat lacté est le lait naturel ou reconstitué, écrémé ou non, ou bien des milieux à base de lait ou de produit d'origine laitière.

Ce substrat peut comporter des éléments couramment utilisés pour la préparation de desserts lactés, éléments solides tels que fruits, pépites de chocolat ou céréales par exemple, mais également des produits sucrés ou chocolatés liquides. La souche selon l'invention présente d'ailleurs l'avantage de résister aux sucres, ce qui permet de fermenter des produits sucrés contenant notamment du saccharose.

La présente invention concerne également les produits lactés fermentés obtenus par la mise en oeuvre de ce procédé, notamment fromages frais fermentés et yoghourts par exemple.

Les produits obtenus par la mise en oeuvre de cette souche présentent une saveur douce et leurs propriétés organoleptiques sont conservées au cours du stockage.

La culture des souches selon l'invention, pures ou en association avec d'autres souches, peut également être utilisée comme probiotique dans l'alimentation humaine ou animale et également comme ferment pour être utilisée dans le procédé selon l'invention.

D'autres caractéristiques et avantages de la présente invention apparaîtront à la lecture des exemples ci-après.

### EXEMPLE 1

### Comparaison de la vitesse d'acidification et de l'activité de post-acidification de différentes souches de s. thermophilus

Un lait écrémé reconstitué à 12 % de matière sèche et enrichi à 0,1 % d'extrait de levure est stérilisé 15 min. à 121°C. Une culture active de *S. thermophilus* comprenant 108 cellules par millilitre est utilisée pour ensemencer ce milieu à 1 % (v/v). Le levain est obtenu après environ 4 heures d'incubation à 44°C. Ce levain est utilisé pour inoculer (1 % v/v) 1 litre de lait écrémé reconstitué à 12 % de matière sèche, enrichi avec 0,1 % d'extrait de levure et préalablement pasteurisé à 95°C pendant 30 min. Le lait inoculé est brassé puis incubé à 44°C. Quand l'acidité atteint un pH de 4,6, la préparation est refroidie 16 heures à 4°C. Ce lait fermenté est soumis à un test de conservation à 8°C pendant 28 jours. Le pH après 28 jours de conservation est comparé pour les différentes souches. Les résultats sont présentés dans le tableau 1. La souche 1-1477 correspond à la souche DN-001 116. Seul le lait fermenté avec la souche *S. thermophilus* DN-001 116 présente une post-acidification quasi nulle après 28 jours de conservation à 8°C. La souche DN-001 116 montre une vitesse d'acidification rapide, on atteint un pH de 4,6 en moins de 4 heures d'incubation à 44°C.

**TABLEAU 1**

| | Code Souche | Temps d'incubation | pH de fin d'incubation | pH à 28 jours | Différence |
|---|---|---|---|---|---|
| 01 | DN-001 003 | 4 h 46 | 4,60 | 4,31 | 0,29 |
| 02 | DN-001 004 | 7 h 21 | 4,62 | 4,26 | 0,36 |
| 03 | DN-001 013 | 7 h 18 | 4,65 | 4,25 | 0,40 |
| 04 | DN-001 015 | 6 h 16 | 4,65 | 4,30 | 0,35 |
| 05 | DN-001 022 | 5 h 18 | 4,60 | 4,15 | 0,45 |
| 06 | DN-001 023 | 3 h 55 | 4,59 | 4,19 | 0,40 |
| 07 | DN-001 025 | 6 h 48 | 4,64 | 4,41 | 0,23 |
| 08 | DN-001 031 | 5 h 43 | 4,60 | 4,40 | 0,20 |
| 09 | DN-001 032 | 4 h 18 | 4,61 | 4,51 | 0,10 |
| 10 | DN-001 047 | 3 h 42 | 4,60 | 4,18 | 0,42 |
| 11 | DN-001 054 | 4 h 19 | 4,60 | 4,16 | 0,44 |
| 12 | DN-001 064 | 5 h 30 | 4,60 | 4,10 | 0,50 |
| 13 | DN-001 067 | 4 h 09 | 4,60 | 4,10 | 0,50 |
| 14 | DN-001 094 | 3 h 54 | 4,61 | 4,08 | 0,53 |
| 15 | DN-001 107 | 3 h 45 | 4,62 | 4,52 | 0,10 |
| 16 | DN-001 111 | 3 h 33 | 4,60 | 4,11 | 0,49 |
| 17 | DN-001 116 | 3 h 45 | 4,61 | 4,58 | 0,03 |
| 18 | DN-001 138 | 4 h 15 | 4,60 | 4,12 | 0,48 |
| 19 | DN-001 143 | 4 h 05 | 4,59 | 4,18 | 0,41 |
| 20 | DN-001 145 | 3 h 40 | 4,60 | 4,17 | 0,43 |
| 21 | DN-001 147 | 6 h 09 | 4,64 | 4,11 | 0,53 |
| 22 | DN-001 156 | 4 h 24 | 4,60 | 4,12 | 0,48 |
| 23 | DN-001 162 | 4 h 05 | 4,60 | 4,14 | 0,46 |
| 24 | DN-001 171 | 5 h 30 | 4,58 | 4,08 | 0,50 |
| 25 | DN-001 181 | 4 h 33 | 4,61 | 4,11 | 0,50 |
| 26 | DN-001 223 | 5 h 30 | 4,60 | 4,21 | 0,39 |
| 27 | DN-001 225 | 3 h 54 | 4,60 | 4,25 | 0,35 |
| 28 | DN-001 228 | 4 h 35 | 4,61 | 4,16 | 0,45 |
| 29 | DN-001 230 | 6 h 15 | 4,64 | 4,20 | 0,44 |
| 30 | DN-001 236 | 5 h 30 | 4,63 | 4,10 | 0,53 |
| 31 | DN-001 242 | 3 h 51 | 4,61 | 4,11 | 0,50 |
| 32 | DN-001 276 | 5 h 25 | 4,60 | 4,18 | 0,42 |
| 33 | DN-001 277 | 3 h 35 | 4,61 | 4,20 | 0,41 |
| 34 | DN-001 280 | 6 h 00 | 4,62 | 4,10 | 0,52 |
| 35 | DN-001 289 | 4 h 50 | 4,61 | 4,06 | 0,55 |
| 36 | DN-001 342 | 5 h 30 | 4,60 | 4,53 | 0,07 |
| 37 | DN-001 343 | 3 h 55 | 4,62 | 4,25 | 0,37 |

### EXEMPLE 2

### Comparaison de l'activité de post-acidification de la souche S. thermophilus 001 116 avec d'autres S. thermophilus

Le levain est préparé comme dans l'exemple 1. Ce levain est utilisé pour inoculer (1 % v/v) 1 litre de lait écrémé reconstitué à 12 % de matière sèche, enrichi avec 0,1 % d'extrait de levure et préalablement pasteurisé 30 min. à 95°C. Le lait inoculé est brassé puis incubé à 4°C. Quand l'acidité atteint 80°D, la préparation est refroidie pendant 16 heures à 4°C. Ce lait fermenté est soumis à un test de conservation à 8°C pendant 28 jours. Le tableau 2 présente la durée de l'incubation et l'évolution du pH et de l'acidité Dornic lors du stockage à 8°C pendant 28 jours.

**TABLEAU 2**

| Code souche | Temps incubation | Acidité Dornic et pH de fin d'incubation | Acidité Dornic et pH à 24 h | Acidité Dornic et pH à 28 jours | Acidité Dornic et différence de pH sur 28 jours |
|---|---|---|---|---|---|
| 001 116 | 3 h 25 | 80°D, 4,80 | 86°D, 4,77 | 92°D, 4,63 | +12°D, -0,17 |
| ST25 | 5 h 30 | 80°D, 4,75 | 86°D, 4,77 | 95°D, 4,57 | +15°D, -0,20 |
| ST44 | 3 h 55 | 80°D, 4,75 | 88°D, 4,71 | 111°D, 4,35 | +21°D, -0,40 |

La post-acidification de la souche ST25 est comparable à la souche DN-001 116 mais sa vitesse d'acidification est beaucoup plus lente. La souche ST44 qui présente une vitesse d'acidification satisfaisante montre une post-acidification très importante.

### EXEMPLE 3

### Stabilité d'un lait ultrafiltré fermenté préparé avec la souche DN 001 116

On prépare un mélange composé d'un rétentat de lait ultrafiltré à 6 % de protéines (NT-NPN x 6,38) et de crème à 40 % de matière grasse. La proportion de chacun des deux constituants est 82 % de rétentat et 18 % de crème. On ajoute de l'hydrolysat de caséine 0,03 % (w/w). La teneur du mélange est de 5,3 % de protéines et de 7,2 % de matière grasse. La préparation est soumise à un préchauffage à 75°C, une homogénéisation à 75°C et à 200 bars, puis à une pasteurisation à 95°C pendant 8 min. avant refroidissement à 40°C. Le mélange est ensuite ensemencé avec une préparation concentrée congelée de la souche DN-001 116 à raison de 30 g/100 1 d'un levain à 9.10⁹ CFU/g. Le mix ensemencé est incubé à 40°C jusqu'à l'obtention d'un pH d'environ 4,7. Après décaillage et soutirage du caillé, celui-ci subit un lissage dans une vanne adaptée avant refroidissement à 20°C dans un échangeur à plaque. Le produit lissé et refroidi est alors conditionné en pot de 1 kg. Ces pots, après palettisation, passent dans un tunnel d'air froid afin de refroidir le produit à 4°C. Le produit est ensuite soumis à un test de conservation à 4°C et à 10°C. Les résultats sont présentés dans le tableau 3.

**TABLEAU 3**

| | Conservation à 4°C | | Conservation à 10°C | |
|---|---|---|---|---|
| Nombre de jours | pH | Dégustation | pH | Dégustation |
| 0 | 4,70 | (+++) | 4,70 | (+++) |
| 3 | 4,66 | (+++) | n.d. | (+++) |
| 12 | 4,66 | (+++) | 4,66 | (+++) |
| 24 | 4,63 | (++) | 4,58 | (++) |

| | | | | |
|---|---|---|---|---|
| (+++) bon, très doux | | | | |
| (++) bon, doux | | | | |

Ces résultats confirment que la souche *S. thermophilus* DN-001 116 présente une post-acidification très réduite à 4°C et à 10°C.

### EXEMPLE 4

### Caractère de résistance aux phages de la souche S. thermophilus DN-001 116

60 phages de *S*. *thermophilus* ont été isolés au cours de 10 années de production et dans des usines de différentes zones géographiques. L'analyse de leur spectre d'hôtes à permis de les classer en 7 sous-groupes. La résistance de la souche DN-001 116 a été testée pour des phages représentatifs de ces 7 sous-groupes (Φ15, Φ57, Φ47, Φ77, Φ76, Φ29, Φ65), le titre des phages excédait 10⁷ Unité Formant Plaques/ml (Les phages soulignés ont été décrits dans la publication Biochimie (1990) 72, 855-862). La souche DN-001 116 est résistante à tous les phages testés.

### EXEMPLE 5

### Résistance aux sucres

On procède comme dans l'exemple 3 mais en ajoutant au milieu à fermenter des quantités variées de saccharose, on mesure le temps pour obtenir le pH cible :
- milieu à 9 % de saccharose : pH 4,7 620 min
- milieu à 13 % de saccharose : pH 4,7 920 min

La souche *Streptococcus thermophilus* a été déposée à la Collection Nationale de Cultures de Microorganismes (CNCM) sous le n° 1-1477 en date du 22 septembre 1994.

## Revendications

1. Souche de *Streptococcus thermophilus* déposée à la Collection Nationale de Cultures de Microorganismes (CNCM) sous le n° I-1477 et ses mutants ayant des caractéristiques d'acidification du lait similaires.

2. Souche de *S. thermophilus* selon la revendication 1 correspondant à la souche n° I-1477.

3. Souche de *S. thermophilus* selon l'une des revendications 1 et 2, caractérisée en ce que les mutants sont obtenus par mutation et sélection de leurs propriétés acidifiantes.

4. Procédé de préparation de produits laitiers fermentés dans lequel un substrat lacté est mis en fermentation avec au moins une souche de *S*. *thermophilus* selon l'une des revendications 1 à 3.

5. Procédé selon la revendication 4, caractérisé en ce que la fermentation est effectuée en présence d'au moins une autre souche de bactérie.

6. Procédé selon la revendication 5, caractérisé en ce que l'autre souche de bactérie est une bactérie lactique.

7. Procédé selon la revendication 6, caractérisé en ce que la bactérie lactique est choisie parmi : *Lactobacillus bulgaricus, Lactobacillus acidophilus, Lactobacillus casei* et *Bifidobacterium.*

8. Procédé selon l'une des revendications 4 à 7, caractérisé en ce que le substrat lacté est du lait.

9. Procédé selon l'une des revendications 4 à 8, caractérisé en ce que le substrat contient des éléments solides.

10. Procédé selon la revendication 9, caractérisé en ce que les éléments solides sont des fruits, des produits chocolatés ou des céréales.

11. Produits fermentés obtenus par la mise en oeuvre du procédé selon l'une des revendications 4 à 10.

## Patentansprüche

1. Streptococcus thermophilus-Stamm, der bei der Collection Nationale de Cultures de Microorganismes (CNCM) unter der Nr. I-1477 hinterlegt wurde, und seine Mutanten, die gleiche Eigenschaften bei der Ansäuerung von Milch aufweisen.

2. S. thermophilus-Stamm nach Anspruch 1, der dem Stamm Nr. I-1477 entspricht.

3. S. thermophilus-Stamm nach einem der Ansprüche 1 und 2, dadurch charakterisiert, daß die Mutanten durch Mutation und Auswahl ihrer acidifizierenden Eigenschaften erhalten werden.

4. Verfahren zur Herstellung von fermentierten Milchprodukten, wobei ein Milchsubstrat mit Hilfe eines S. thermophilus-Stamms nach einem der Ansprüche 1 bis 3 der Fermentation unterworfen wird.

5. Verfahren nach Anspruch 4, dadurch charakterisiert, daß die Fermentation in Gegenwart von mindestens einem weiteren Bakterienstamm durchgeführt wird.

6. Verfahren nach Anspruch 5, dadurch charakterisiert, daß der andere Bakierienstamm ein Milchsäurebakterium ist.

7. Verfahren nach Anspruch 6, dadurch charakterisiert, daß das Milchsäurebakterium ausgewählt wird aus: Lactobacillus bulgaricus, Lactobacillus acidophilus, Lactobacillus casei und Bifidobacterium.

8. Verfahren nach einem der Ansprüche 4 bis 7, dadurch charakterisiert, daß das Milchsubstrat Milch ist.

9. Verfahren nach einem der Ansprüche 4 bis 8, dadurch charakterisiert, daß das Substrat feste Bestandteile enthält.

10. Verfahren nach Anspruch 9, dadurch charakterisiert, daß die festen Bestandteile Früchte, Schokoladenprodukte oder Getreide sind.

11. Fermentierte Produkte, die durch Durchführung des Verfahrens nach einem der Ansprüche 4 bis 10 erhalten werden.

## Claims

1. *Streptococcus thermophilus* strain deposited at the Collection Nationale de Cultures de Microorganismes (CNCM) under No. I-1477 and mutants thereof which have similar milk acidification characteristics.

2. *S*. *thermophilus* strain according to Claim 1, which corresponds to strain No. I-1477.

3. *S. thermophilus* strain according to either of Claims 1 and 2, characterized in that the mutants are obtained by mutation and selection of their acidifying properties.

4. Process for the preparation of fermented dairy products in which a milk substrate is fermented with at least one *S*. *thermophilus* strain according to one of Claims 1 to 3.

5. Process according to Claim 4, characterized in that the fermentation is carried out in the presence of at least one other strain of bacterium.

6. Process according to Claim 5, characterized in that the other strain of bacterium is a lactic acid bacterium.

7. Process according to Claim 6, characterized in that the lactic acid bacterium is chosen from: *Lactobacillus bulgaricus, Lactobacillus acidophilus, Lactobacillus casei* and *Bifidobacterium.*

8. Process according to one of Claims 4 to 7, characterized in that the milk substrate is milk.

9. Process according to one of Claims 4 to 8, characterized in that the substrate contains solid items.

10. Process according to Claim 9, characterized in that the solid items are fruits, chocolate products or cereals.

11. Fermented products obtained using the process according to one of Claims 4 to 10.
